# EUROPEAN PATENT SPECIFICATION

(11) **EP 3 610 860 B1**
(45) Date of publication and mention of the grant of the patent: **23.09.2026**
(21) Application number: 18000676.9
(22) Date of filing: 15.08.2018
(51) Int. Cl.: A61P 11/00, A61K 9/16, A61K 9/50, A61K 33/00

(54) **COMPOSITION OF CARBON DIOXIDE ABSORBENT OR ADSORBENT, WHEREBY THE SAID COMPOSITION CONTAINS A POLYMERIC COATING SELECTED FROM SILICONE RUBBER AND CELLULOSE DERIVATIVES**
ZUSAMMENSETZUNG AUS KOHLENDIOXIDABSORPTIONSMITTEL ODER -ADSORPTIONSMITTEL, WOBEI DIE BESAGTE ZUSAMMENSETZUNG EINE AUS SILIKONKAUTSCHUK UND CELLULOSE DERIVATE AUSGEWÄHLTE POLYMERBESCHICHTUNG ENTHÄLT
COMPOSITION D'ABSORBANT OU D'ADSORBANT DE DIOXYDE DE CARBONE, LADITE COMPOSITION CONTENANT UN REVÊTEMENT POLYMÈRE SÉLECTIONNÉ À PARTIR DE CAOUTCHOUC DE SILICONE ET DE DÉRIVÉS CELLULOSIQUES

(43) Date of publication of application: 19.02.2020
(73) Proprietor: O11 biomedical GmbH, 52074 Aachen (DE)
(72) Inventor: Jockenhövel, Stefan, 52074 Aachen (DE); Cornelissen, Christian, 52072 Aachen (DE); Spillner, Jan, 40476 Düsseldorf (DE); Kok, Robbert Jan, 3438CS Nieuwegein (NL)
(74) Representative: Viering, Jentschura & Partner mbB Patent- und Rechtsanwälte

(56) References cited:
- EP-B1- 1 313 555
- WO-A2-2005/097075
- WO-A2-2010/048478
- US-A- 4 963 327
- US-A1- 2011 142 905
- US-A1- 2015 245 999
- US-A1- 2017 319 617
- MIYAGUCHI NAOYUKI ET AL: "Transintestinal Systemic Oxygenation Using Perfluorocarbon", SURGERY TODAY, SPRINGER, JP, vol. 36, no. 3, 1 March 2006 (2006-03-01), pages 262 - 266, XP037139843, ISSN: 0941-1291, DOI: 10.1007/S00595-005-3135-Z

## Description

The instant invention discloses a composition of 10 wt% to 99 wt% of carbon dioxide absorbent or adsorbent, whereby the composition contains 1 wt% to 90 wt%, relative to the total composition, of a polymeric coating selected from the group consisting of silicone rubber obtained from liquid silicone rubber and cellulose for use in the prophylaxis and treatment of respiratory diseases under conditions where an excess of carbon dioxide is present in the gastrointestinal tract of a mammal, for the absorbance of carbon dioxide from the gastrointestinal tract of a mammal.

The absorption/removal of carbon dioxide is a mandatory fact in breathing systems of mammals including ungulates, carnivores and humans already without suffering from a disease.

Chronic airway disease is the third most frequent cause of mortality worldwide [WHO: The global burden of non-communicable diseases. 2014], with chronic obstructive pulmonary disease (COPD) representing the major share of 4.2 million deaths in 2008. Patients developing chronic *hypercapnia* (accumulation of carbon dioxide) have a chance of 90% to die within 5 years (Costello R, Deegan P, Fitzpatrick M, McNicholas WT. Am J Med 1997; 103:239.) These patients also experience a markedly reduced quality of life, suffering from constant *dyspnea. The European Lung White Book* notes a high economic burden of COPD: 23 billion € of direct health care costs are estimated for the EU yearly with an additional 25 billion € due to loss of productivity.

Standard therapy of patients with chronic hypercapnic failure is mechanical ventilation via a nasal or a full-face mask to relieve symptoms and improve prognosis. This therapy restricts patients' mobility and impedes communication and societal participation. Extracorporeal carbon dioxide Removal (ECCO2R) is no option for patients with, chronic diseases due to problems regarding chemo- and biocompatibility.

Furthermore, the technical need is extremely high requiring an intensive care.

### Prior Art

*Ex vivo* systems during narcosis use either sodalime based on 80 wt% calcium hydroxide, 5 wt% sodium hydroxide, remnant water provided by Drägerwerk, Germany, Amsorb^{®}, based on 70 wt% calcium hydroxide, 0.7 wt% calcium chloride, remnant water, or Baralyme^{®} based on 80 wt% of calcium hydroxide and 20 wt% barium hydroxide, respectively, as carbon dioxide absorbers during medical use. However, those systems are highly caustic thus not pharmaceutically acceptable for oral intake.

WO2008073517 = US2012/0053329A1, abandoned 2015 discloses oral use of bismuth subnitrate. This substance may be used for the treatment of excessive gas production, for the prevention and treatment of traveler's diarrhea, to reduce the symptoms of microscopic colitis and to induce remission of this condition, for the medical management of abdominal bloating and diarrhea associated with functional bowel disorder such as irritable bowel syndrome and as adjunctive initial or maintenance therapy for inflammatory bowel disease [017, 018].

CN101905001A1, deemed to be withdrawn 2013, claims an oral drug for removing gastrointestinal gas from changing the Doppler acoustic impedance, characterized by 5 kind of drugs, including Amomum villosum, citrus, *Citrus aurantium,* betel nut and radish seed, preferably 10 g of Amomum villosum, 12 g of wood, 12 g of *Citrus aurantium,* 15 g of betel nut and 15 g of radish seed.

US5 922 317A1, expired 2007 or EP1 085 923A4, deemed to be withdrawn 2004, discloses a bacterial pharmaceutical composition for treatment of a diver during decompression consisting of an effective amount of a hydrogenase bacteria selected from the group consisting of *Methanobrevi bacter* and *Alcaligenes* in a pharmaceutically acceptable carrier which is capable of delivering the bacteria to the large intestine of the diver, wherein said composition is formulated as a delayed release capsule or an enteric-coated composition.

Miagutchi, N, Nagahiru, I, Kotani, K, Nakanishi, H, Mori, H, Osagari, T, Shimizu, N, Surg. Today (2006) 36: 2002 - 2006, Transintestinal Systemic Oxygenation using perfluorocarbon proposes perfluorocarbon FC-77 from Sumitomo 3-M as means for carbon dioxide removal.

WO 2005/097075 A2 describes a tamper resistant dosage form comprising an adsorbent and an adverse agent, wherein the dosage form may furthermore comprise a hydrophobic material disposed at least on a portion of the outer surface of the adsorbent. The disclosed dosage form may preferably be used to avoid abuse of opioids.

US 2015/0245999 A1 describes the use of activated carbon to adsorb unspecified substances from the gastrointestinal tract, wherein the activated carbon forms a core that is surrounded by a first layer comprising an insoluble semipermeable membrane that allows for diffusion of ions and molecules. The formulation is used for treatment of gastrointestinal diseases and/or dysfunctions or malfunction of the gastrointestinal tract.

US 2017/319617 A1 describes a method for treating a condition selected from the group consisting of proctitis, radiation proctitis, and pouchitis using a formulation comprising a core of activated carbon and a first layer comprising an insoluble semipermeable membrane, wherein the first layer surrounds the core. The semipermeable membrane allows for diffusion of ions and molecules.

WO 2010/048478 A2 describes a multilayer modular release system for drug-delivery comprising different layers. By adjusting different parameters of each individual layer, such as drug concentration, it is possible to a establish a variety of drug release profiles.

US 2011/142905 A1 describes a pharmaceutical composition for controlled delivery of at least one active ingredient into an aqueous phase.

All prior art solutions to absorb carbon dioxide from the mammal body are either caustic or toxic *in vivo,* use bacterial drugs or other drugs interacting with the mammal body.

The present invention proposes the removal of carbon dioxide from the mammal body with a means that will not release drugs into the body but will simply and effectively decrease carbon dioxide concentration by way of physical absorption or adsorption, respectively.

The instant invention provides a composition of 10 wt% to 99 wt% of carbon dioxide absorbent or adsorbent, wherein the carbon dioxide absorbent or adsorbent is selected from the group consisting of inorganic hydroxides of alkali hydroxides and/or alkaline earth metal hydroxides, molecular sieves, zeolites, zinc (ii) ions, deep eutectic solvents and/or silica for use in the prophylaxis and treatment of respiratory diseases under conditions where an excess of carbon dioxide is present in the gastrointestinal tract of a mammal, characterized in that the composition contains 1 wt% to 90 wt%, relative to the total composition, of a polymeric coating selected from the group consisting of silicone rubber obtained from liquid silicone rubber and cellulose derivative, wherein the cellulose derivatives are selected from the group consisting of cellulose ester, regenerated cellulose and a water insoluble cellulose ether.

According to a preferred embodiment of the instant invention the composition comprises 50 wt% to 99 wt%, of a carbon dioxide absorbent or adsorbent, whereby the composition contains 1 wt% to 50 wt%, relative to the total composition, of a polymeric coating selected from the group consisting of silicone rubber obtained from liquid silicone rubber and the claimed cellulose derivatives.

According to a further preferred embodiment of the instant invention the carbon dioxide absorbent or adsorbent is active carbon.

Examples of inorganic hydroxides of alkali hydroxides and / or alkaline earth metal hydroxides are lithium hydroxide, sodium hydroxide, potassium hydroxide, rubidium hydroxide, cesium hydroxide, beryllium hydroxide, magnesium hydroxide, calcium hydroxide, strontium hydroxide and barium hydroxide, wherein sodium hydroxide, calcium hydroxide and/or barium hydroxide being preferred.

A molecular sieve is a material with pores (very small holes) of uniform size. According to IUPAC notation, microporous materials have pore diameters of less than 2 nm and macroporous materials have pore diameters of greater than 50 nm; the mesoporous category thus lies in the middle with pore diameters between 2 and 50 nm. Microporous materials may be Zeolite or active carbon. An example of molecular sieves are type 4A and type 5A molecular sieves obtained from Zeochem AG, Switzerland.

Zeolites are microporous, aluminosilicate minerals commonly used as commercial adsorbants and catalysts which can be also obtained by artificial synthesis. Examples of zeolites are Sperasorb^{®} obtained from Intersurgical USA.

Example of zinc (ii) ions is zinc peroxide obtained from Sigma Aldrich.

Deep eutectic solvents are systems formed from a eutectic mixture of Lewis or Brønsted acids and bases which can contain a variety of anionic and/or cationic. Examples of deep eutectic solvents are monoethanolamine hydrochloride, ethylenediamine obtained from Sigma Aldrich.

Example of Silica is KC type silica gel obtained from Kali-Chemie AG.

According to a further preferred embodiment of the instant invention the carbon dioxide absorbent or adsorbent is either in granular form having a diameter of 0.1 mm to 10 mm, preferably 2 mm to 4 mm of US Pharmacopeia 26 or being pulverized in a *per* se known manner using e.g. pistil and mortar or a ball mill or wherein the carbon dioxide absorbent is in a liquid form.

Examples of carbon dioxide absorbent in a liquid form are those using deep eutectic solvents, preferably a combination of a non-toxic salt and a non-toxic hydrogen bond donor *e.g*. choline chloride as a salt and urea as a hydrogen bond donor.

According to a further preferred embodiment of the instant invention the silicone rubber is obtained from a liquid silicone rubber, wherein said liquid rubber being vulcanized, preferably either as a two-part, platinum-catalyzed liquid silicone rubber or a one-part liquid silicone rubber with peroxide initiator(s).

The liquid silicone rubber always comprises a principal silicone oxygen chain (the siloxane backbone) and an organic moiety bound to the silicone. The properties of silicone rubber vary greatly depending on the organic groups and the chemical structure.

The organic groups may be methyl, vinyl, phenyl, trifluoropropyl or other groups. Depending on which organic groups are present, silicone polymers in common use are classified as follows: Polydimethylsiloxane (PDMS or MQ, respectively), denotes a polymer in which two methyl groups are bound to the siloxane backbone, VPDMS or VMQ, respectively, stands for polydimethylsiloxane in which a small number of methyl groups have been replaced by vinyl groups, PVMQ stands for an VMQ in which a small number of methyl groups have been replaced by phenyl groups and FVMQ stands for an VMQ in which a small number of methyl groups have been replaced by trifluoropropyl substituents.

Examples of silicone rubber is obtained from a liquid silicone rubber (LSR), wherein said liquid rubber being vulcanized, as a two-part, platinum-catalyzed liquid silicone rubber, many of them being approved for limited exposure, *i.e*. for one day or less as medical grade LSR or biomedical grade silicone elastomer = silicone rubber.

They are available as room-temperature vulcanizing 2-component type (RTV-2) silicone rubber from Wacker Chemie AG as Elastosil^{®} M type compounds *e.g.* Elastosil M 4601 A/B or from DOW Consumer Solutions as *Silastic*^{®} Q7 47XX series BioMedical Grade Liquid Silicone Rubbers Parts A and B, *e.g.* Q7 4720 - Q7 4780, or Dow Corning C6-1XX series BioMedical Grade Liquid Silicone Rubbers Parts A and B, *e.g.* C6-135 - C6-180.

Examples of silicone rubber is obtained from a liquid silicone rubber, wherein said liquid rubber being vulcanized, as one-part liquid silicone rubber with peroxide initiator(s) within minutes at a temperature between 100 and 200 °C.

They are available as peroxide curing 1 part silicone rubber curing within 10 minutes at about 150 °C to 200 °C from Wacker Chemie AG as Elastosil^{®}A, Elastosil^{®}E, Elastosil^{®}N or Elastosil^{®}R type compounds or from DOW Consumer Solutions as *Silastic*^{®} Q7 45XX series BioMedical Grade 1 part Silicone elastomers with peroxide initiator, *e.g.* Q7 4535 to Q7 4565, or Dow Corning C6-2XX series BioMedical Grade 1 part Silicone elastomers with peroxide initiator, e.g. C6-235 to C6-265.

According to the instant invention the cellulose derivatives are selected from the group consisting of cellulose ester, regenerated cellulose and/or a water insoluble cellulose ether.

Examples of cellulose esters are cellulose acetate, cellulose acetate butyrate, cellulose acetate propionate, cellulose palmitate, cellulose propionate, cellulose stearate and cellulose triacetate.

Examples of regenerated Cellulose and its enteral use is described in dissertation of Mr. Bhavik J Bhatt, Regenerated cellulose for controlled oral drug delivery 2012, first published as WO2011/057206 in 2012 and only granted as US9,050,264B2 in 2015.

So cellulose is converted into a methylolcellulose solution by reacting cotton linter with paraformaldehyde in anhydrous DMSO under elevated temperature. Capsule shells were fabricated by dipping the Teflon pins of dimensions 8.0 mm x 10 cm (capsule body) and 8.3 mm x 10 cm (capsule cap) in the methylolcellulose solution, and subsequently rotating the pins in air. The coated pin was placed in an excess of acetone for some minutes and then air dried. The dipping and rotating steps were repeated. The coated Teflon pins were then immersed in deionized water for about 24 h. The capsule shells were then cut to the desired length and cured at elevated temperature. After curing, the shells were removed from the pins, and the 2 part capsule stored under dry conditions. Said regenerated cellulose is also available for sale as Spectra/Por # 132,645 obtained from Spectrum Labs, Germany.

Examples of cellulose ethers are benzyl cellulose and ethyl cellulose.

According to the instant invention the composition is for use for the absorbance of carbon dioxide from the gastrointestinal tract of a mammal, under conditions where an excess of carbon dioxide is present in the gastrointestinal tract of a mammal, for the prophylaxis and treatment of respiratory diseases.

Examples of respiratory diseases are acute and chronic airway diseases such as adult respiratory distress syndrome, asthma and mucoviscidosis, including chronic obstructive pulmonary disease (COPD), wherein treatment of chronic obstructive pulmonary disease (COPD) being preferred.

According to a further preferred embodiment of the instant invention the composition is used per oral administration at least one, preferably at least 3 times per day.

According to a further preferred embodiment of the instant invention the oral administration is performed with a tablet, wherein carbon dioxide absorbent or adsorbent is embedded in silicone rubber or with a capsule, wherein the carbon dioxide absorbent or adsorbent is contained in a capsule made of vulcanized silicone rubber being preferably sealed with a silicone rubber coating or of a water insoluble cellulose, preferably sealed.

In accordance with the instant invention tablets are a solid unit dosage prepared either by molding, by compression, by cutting from larger units or by embedding carbon dioxide particles absorbent or adsorbent in silicone rubber or in water insoluble cellulose. It comprises a mixture of components, processed from the components into a solid dose.

In accordance with the instant invention a capsule refers either to hard-shelled capsules which contains powdered or pelletized ingredients made in two halves, a smaller diameter body that is filled and then sealed using a larger diameter cap, and further sealed, if necessary. Alternatively, in case of soft-shelled one-part capsules are used for liquid ingredients. Standard sizes of two-piece capsules for oral intake go from size 5 (0.13 ml) to size 000 (1.36 ml).

According to a further preferred embodiment of the instant invention the oral dosage form is a tablet, wherein the tablet is selected from granules, mini-tablets and tablets having a weight of 0.01 µg to 0.6 g, preferably 0.01 to 0.1 g.

Mini-tablets in accordance with the instant invention have a diameter of 2 mm to 1 mm and can be produced in a per se known manner, see *e.g.* review article https://www.americanpharmaceuticalreview.com/Featured-Articles/190921-Minitablets-Manufacturing-Characterization-Methods-and-Future-Opportunities/.

Granules in accordance with the instant invention have a diameter of 2 mm or less and can be produced in a *per se* known manner wherein the carbon dioxide absorbent or adsorbent is embedded in silicone rubber granules. These granules have a weight of 0.01 µg to 0.6 g, preferably 0.01 g to 0.1 g.

According to a further preferred embodiment of the instant invention the oral administration is given to a mammal which mammal is selected from ungulates including horses and camels, carnivores including cats, dogs and humans, wherein humans are preferred.

An ungulate or hoofed mammal is a mammal of the order *ungulate,* which comprises odd-toed *ungulates* including horses, rhinos and tapirs and even-toed *ungulates* including cows, sheep, goats, buffalos, deer, giraffes, pigs and camels. Of these horses and camels being preferred.

A carnivore is a mammal of the order *carnivora,* which comprises the cats, dogs, bears, hyenas, weasels, civet cats, racoons, and mongooses, wherein cats and dogs being preferred.

Of the above mentioned mammals humans are the most preferred.

The present invention is now illustrated by working and application examples.

### Galenic Example 1 (tablet with sodalime embedded in silicone rubber)

40 g granular carbon dioxide absorbent sodalime (Drägersorb 800+, with about 78 wt% to 84 wt-% calcium hydroxide, 2 wt% - 4 wt% sodium hydroxide, about 14 wt% - 18 wt% water, about 0.1 wt% indicator ethyl violett, size 2 to 4 mm USP 27, obtained from Drägerwerk AG & Co KgaA) was dip coated into 40 g of RTV-2 silicone rubber (Elastosil^{®} M 4601 A/B, using a platinum catalyst for room temperature curing, obtained from Wacker Chemie AG). After curing for 12 h the mass is processed into tablet-shaped bodies having a weight of about 0.5 g.

### Galenic Example 2 (tablet with pulverized sodalime embedded in silicone rubber)

Galenic example 1 is repeated but using granular carbon dioxide absorbent sodalime being pulverized using a mortar and then being processed with RTV-2 silicone rubber (Elastosil^{®} M 4601 A/B) as above forming tablet-shaped bodies having a weight of about 0.5 g

### Galenic Example 3 (CO₂ permeable regenerated cellulose or adsorbent capsule filled with sodalime)

A capsule made of regenerated cellulose was produced from dialysis membranes (Spectra/Por 132645 obtained from Spectrum labs, Germany) having a volume of 0.60 ml, weight 0.1 g, was filled with 0.4 g pulverized sodalime of Drägersorb 800+, as defined in galenic example 2.

### Evaluation of carbon dioxide absorption by tablet with sodalime embedded in silicone rubber in simulated intestinal fluid

Simulated intestinal fluid (SIF) without enzyme was produced according to US pharmacopeia 26 by dissolving 6.805 g potassium dihydrogen phosphate and 0.896 g sodium hydroxide (both obtained from SigmaAldrich, Germany) in 1 L of distilled water. SIF enriched with carbon dioxide in a bubble process and pH was adjusted to 6.8 using a pH meter (HANNA instruments, Germany) and a 5M solution of sodium hydroxide (SigmaAldrich, Germany).

15 ml test tubes (Sarstedt, Germany) were completely filled with carbon dioxide enriched SIF. Either a tablet of 0.5 g with sodalime according to galenic example 1, 2 or 3 or 0.5 g of pure silicone granules produced from platinum cured silicone acquired from Wacker Chemie AG, (Elastosil M4601 A/B) were added to the SIF. Pure carbon dioxide enriched SIF served as a control. Carbon dioxide partial pressure, pH and hydrogen carbonate (HCO₃) concentration were measured after 0 min, 10 min, 1 h, 2 h, 12 h using a blood gas analyser (Radiometer ABL 800 Flex). For each experiment, 3 separate test tubes were prepared for each material (tablet with sodalime according to galenic example 1, 2 and 3, silicone rubber tablets, pure SIF).

The following tables show the timeline of the carbon dioxide decrease and the development of pH-changes within 12 h after application.

**Table 1: Galenic example 1 & 2 [tablets]**

| | | 0 min | 10 min | 1 h | 2 h | 12 h |
|---|---|---|---|---|---|---|
| Pure SIF | Mean pCO2 | 69.1 | 70 | 70.4666667 | 69.5666667 | 66.7 |
| | SD pCO2 | 0,49665548 | 1,02306728 | 0,57927157 | 1,07806411 | 0,66833126 |
| | Mean | | | | | |
| | HCO3 | 9.63333333 | 9.8 | 9.96666667 | 9.9 | 9.63333333 |
| | SD HCO3 | 0,04714045 | 0,08164966 | 0,04714045 | 0,08164966 | 0,04714045 |
| | Mean pH | 6.778 | 6.78 | 6.784 | 6.786 | 6.79233333 |
| | SD pH | 0,00244949 | 0,00326599 | 0,00244949 | 0,00294392 | 0,00286744 |
| Silicone Rubber tablets | Mean pCO2 | | 69,6333333 | 67,8 | 66,5666667 | 64,9666667 |
| | SD pCO2 | | 0,601849 | 0,29439203 | 1,07806411 | 0,49216077 |
| | Mean | | | | | |
| | HCO3 | | 9.8 | 9.73333333 | 9.63333333 | 9.4 |
| | SD HCO3 | | 0,08164966 | 0,04714045 | 0,12472191 | 0 |
| | Mean pH | | 6.78166667 | 6.78933333 | 6.793 | 6.79366667 |
| | SD pH | | 0,0004714 | 0,0004714 | 0,00216025 | 0,00188562 |
| **Galenic Example 2** | Mean pCO2 | | 56.8 | 37.1333333 | 32.5333333 | 5 |
| | SD pCO2 | | 0,98994949 | 4,85615303 | 3,27142511 | 0 |
| | Mean | | | | | |
| | HCO3 | 9.53333333 | 8.86666667 | 8.46666667 | 4.3 | |
| | SD HCO3 | 0,0942809 | 0,20548047 | 0,18856181 | 0,24494897 | |
| | Mean pH | 6.85566667 | 7.01166667 | 7.04666667 | 7.61633333 | |
| | SD pH | 0,00418994 | 0,04606035 | 0,035046 | 0,16160927 | |
| **Galenic Example 1** | Mean pCO2 | 68.5666667 | 61.5 | 60.4666667 | 33.8666667 | |
| | SD pCO2 | 0,80138769 | 1,25698051 | 1,74419673 | 4,52425562 | |
| | Mean | | | | | |
| | HCO3 | 9.8 | 9.23333333 | 9.26666667 | 6.93333333 | |
| | SD HCO3 | 0,08164966 | 0,12472191 | 0,04714045 | 0,4027682 | |
| | Mean pH | 6.78733333 | 6.81066667 | 6.819 | 6.94466667 | |
| | SD pH | 0,00249444 | 0,0046428 | 0,00848528 | 0,04823784 | |

### Results

The produced tablet with granulized sodalime according to galenic example 1 led to a slow absorption kinetic of CO₂ from the SIF. Thus, pH changes were well within limits tolerable for an *in vivo* application. There is a decrease of carbon dioxide of about 12 % after 2 h and of about more than 50 % after 12 h. There is a decrease of carbon dioxide of about 42,3 % after 2 h and of about more than 91 % after 12 h in galenic example 2 using pulverized sodalime.

**Table 2: Galenic example 3 [capsule]**

| | | 0 min | 10 min | 1 h | 2 h | 12 h |
|---|---|---|---|---|---|---|
| **Pure SIF** | Mean pCO2 | 53 | 51.3333333 | 51.3333333 | 48.6666667 | 48 |
| | SD pCO2 | 0 | 0,94280904 | 0,47140452 | 1,24721913 | 1,63299316 |
| | Mean | | | | | |
| | HCO3 | 7.2 | 7 | 7 | 6.83333333 | 6.7 |
| | SD HCO3 | 0 | 0,14142136 | 0,08164966 | 0,12472191 | 0,16329932 |
| | Mean pH | 6.76733333 | 6.77166667 | 6.771 | 6.77866667 | 6.782 |
| | SD pH | 0,00821922 | 0,0062361 | 0,00141421 | 0,00410961 | 0,00535413 |
| **Galenic Example 3** | **Mean pCO2** | | 43.0 | 32.3333333 | 23.3333333 | 6.23333333 |
| | SD pCO2 | | 3.55902608 | 6.64997911 | 4.49691252 | 0.67986927 |
| | Mean | | | | | |
| | HCO3 | | 7 | 7.13333333 | 7.2 | 7.3 |
| | SD HCO3 | | 0,08164966 | 0,41096093 | 0,6164414 | 0,28284271 |
| | Mean pH | | 6.846 | 6.98033333 | 7.12233333 | 7.72933333 |
| | SD pH | | 0.04176921 | 0.12082034 | 0.12574136 | 0.09527971 |

### Results

The produced capsule with pulverized sodalime according to galenic example 3 led to a fast absorption kinetic of CO₂ from the SIF. There is a decrease of carbon dioxide of about 18 % after 2 h and of about more than 85 % after 12 h in galenic example 3 using pulverized sodalime. The fast absorption kinetic led to a pH shift into alkaline levels.

### Note:

The differences in speed of CO₂ absorption from the SIF can be explained by different diffusion distances through the silicone or cellulose shells. In galenic example 1, dip-coating the sodalime granules led to the longest diffusion distance, resulting in slow CO₂ absorption. The shorter diffusion distances in galenic examples 2 and 3 explain a quicker CO₂ absorption.

## Claims

1. Composition of 10 wt% to 99 wt% of carbon dioxide absorbent or adsorbent, wherein the carbon dioxide absorbent or adsorbent is selected from the group consisting of inorganic hydroxides of alkali hydroxides and/or alkaline earth metal hydroxides, molecular sieves, zeolites, zinc (ii) ions, deep eutectic solvents and/or silica for use in the prophylaxis and treatment of respiratory diseases in a mammal under conditions where an excess of carbon dioxide is present in the gastrointestinal tract of a mammal, for the absorbance of carbon dioxide from the gastrointestinal tract of a mammal, **characterized in that** the composition contains 1 wt% to 90 wt%, relative to the total composition, of a polymeric coating selected from the group consisting of silicone rubber obtained from liquid silicone rubber and cellulose derivative, wherein the cellulose derivatives are selected from the group consisting of cellulose ester, regenerated cellulose and a water insoluble cellulose ether.

2. The composition of claim 1 having 50 wt% to 99 wt%, of a carbon dioxide absorbent or adsorbent, whereby the composition contains 1 wt% to 50 wt%, relative to the total composition, of a polymeric coating.

3. The composition of any of claims 1 to 2, wherein carbon dioxide absorbent or adsorbent is either in granular form having a diameter of 0.1 to 10 mm, preferably 2 to 4 mm of US Pharmacopeia 26 or being pulverized in a *per* se known manner or wherein the carbon dioxide absorbent or adsorbent is in a liquid form.

4. The composition of any of claims 1 or 2, wherein the silicone rubber is obtained from a liquid silicone rubber wherein said liquid rubber being vulcanized, preferably either as a two-part, platinum-catalyzed liquid silicone rubber or as a one-part liquid silicone rubber with peroxide initiator(s).

5. The composition of any of claims 1 to 4, wherein the composition is used per oral administration at least one, preferably at least 3 times per day.

6. The composition of any of claims 1 to 5, wherein the oral administration is performed with a tablet, wherein carbon dioxide absorbent or adsorbent is embedded in silicone rubber or with a capsule, wherein the carbon dioxide absorbent or adsorbent is contained in a capsule made of vulcanized silicone rubber being preferably sealed with a silicone rubber coating or of a water insoluble cellulose.

7. The composition of claim 6, wherein the tablet is selected from granules, mini-tablets and tablets having a weight of 0.01 µg to 0.6 g, preferably 0.01 g to 0.1 g.

8. The composition of any of claims 1 to 7, wherein the oral administration is given to a mammal which is selected from ungulates including horses and camels, carnivores including cats, dogs and humans, wherein humans being preferred to mammals.

## Patentansprüche

1. Zusammensetzung von 10 Gew.-% bis 99 Gew.-% Kohlendioxid-Absorptionsmittel oder -Adsorptionsmittel, wobei das Kohlendioxid-Absorptionsmittel oder -Adsorptionsmittel ausgewählt ist aus der Gruppe bestehend aus anorganischen Hydroxiden von Alkalihydroxiden und/oder Erdalkalimetallhydroxiden, Molekularsieben, Zeolithen, Zink(II)-Ionen, stark eutektischen Lösungsmitteln und/oder Siliciumdioxid zur Verwendung bei der Prophylaxe und Behandlung von Atemwegserkrankungen bei einem Säuger unter Bedingungen, bei denen ein Überschuss an Kohlendioxid im Magen-DarmTrakt eines Säugers vorhanden ist, für die Absorption von Kohlendioxid aus dem Magen-Darm-Trakt eines Säugers, **dadurch gekennzeichnet, dass** die Zusammensetzung 1 Gew.- % bis 90 Gew.-%, bezogen auf die Gesamtzusammensetzung, einer polymeren Beschichtung enthält, ausgewählt aus der Gruppe bestehend aus Silikonkautschuk, erhalten aus flüssigem Silikonkautschuk, und Cellulosederivat, wobei die Cellulosederivate ausgewählt sind aus der Gruppe bestehend aus Celluloseester, regenerierter Cellulose und einem wasserunlöslichen Celluloseether.

2. Zusammensetzung nach Anspruch 1 mit 50 Gew.-% bis 99 Gew.-% eines Kohlendioxid-Absorptionsmittels oder -Adsorptionsmittels, wobei die Zusammensetzung 1 Gew.-% bis 50 Gew.-% einer polymeren Beschichtung, bezogen auf die Gesamtzusammensetzung, enthält.

3. Zusammensetzung nach einem der Ansprüche 1 bis 2, wobei das Kohlendioxid-Absorptionsmittel oder -Adsorptionsmittel entweder in Granulatform mit einem Durchmesser von 0,1 bis 10 mm, vorzugsweise 2 bis 4 mm, von US Pharmacopeia 26 vorliegt oder in *an* sich bekannter Weise pulverisiert ist oder wobei das Kohlendioxid-Absorptionsmittel oder -Adsorptionsmittel in flüssiger Form vorliegt.

4. Zusammensetzung nach einem der Ansprüche 1 oder 2, wobei der Silikonkautschuk aus einem flüssigen Silikonkautschuk erhalten wird, wobei der flüssige Kautschuk vulkanisiert ist, vorzugsweise entweder als zweiteiliger, platinkatalysierter flüssiger Silikonkautschuk oder als einteiliger flüssiger Silikonkautschuk mit Peroxidinitiator(en).

5. Zusammensetzung nach einem der Ansprüche 1 bis 4, wobei die Zusammensetzung für die orale Verabreichung mindestens einmal, vorzugsweise mindestens dreimal pro Tag, verwendet wird.

6. Zusammensetzung nach einem der Ansprüche 1 bis 5, wobei die orale Verabreichung mit einer Tablette durchgeführt wird, wobei das Kohlendioxid-Absorptionsmittel oder - Adsorptionsmittel in Silikonkautschuk eingebettet ist, oder mit einer Kapsel, wobei das Kohlendioxid-Absorptionsmittel oder -Adsorptionsmittel in einer Kapsel aus vulkanisiertem Silikonkautschuk, der vorzugsweise mit einer Silikonkautschukbeschichtung versiegelt ist, oder aus einer wasserunlöslichen Cellulose enthalten ist.

7. Zusammensetzung nach Anspruch 6, wobei die Tablette aus Granulaten, Minitabletten und Tabletten mit einem Gewicht von 0,01 µg bis 0,6 g, vorzugsweise 0,01 g bis 0,1 g, ausgewählt ist.

8. Zusammensetzung nach einem der Ansprüche 1 bis 7, wobei die orale Verabreichung an einem Säugetier erfolgt, das aus Huftieren, einschließlich Pferden und Kamelen, Karnivoren, einschließlich Katzen, Hunden und Menschen, ausgewählt ist, wobei als Säugetiere Menschen vorzuziehen sind.

## Revendications

1. Composition de 10 % en poids à 99 % en poids d'absorbant ou d'adsorbant de dioxyde de carbone, dans laquelle l'absorbant ou l'adsorbant de dioxyde de carbone est choisi dans le groupe constitué d'hydroxydes inorganiques d'hydroxydes alcalins et/ou d'hydroxydes de métaux alcalino-terreux, de tamis moléculaires, de zéolites, d'ions zinc (ii), de solvants eutectiques profonds et/ou de silice pour une utilisation dans la prophylaxie et le traitement de maladies respiratoires chez un mammifère dans des conditions où un excès de dioxyde de carbone est présent dans le tractus gastro-intestinal d'un mammifère, pour l'absorbance de dioxyde de carbone provenant du tractus gastro-intestinal d'un mammifère, **caractérisée en ce que** la composition contient 1 % en poids à 90 % en poids, par rapport à la composition totale, d'un revêtement polymère choisi dans le groupe constitué de caoutchouc de silicone obtenu à partir de caoutchouc de silicone liquide et de dérivé de cellulose, dans laquelle les dérivés de cellulose sont choisis dans le groupe constitué d'ester de cellulose, de cellulose régénérée et d'un éther de cellulose insoluble dans l'eau.

2. Composition selon la revendication 1 ayant 50 % en poids à 99 % en poids d'un absorbant ou d'un adsorbant de dioxyde de carbone, moyennant quoi la composition contient 1 % en poids à 50 % en poids, par rapport à la composition totale, d'un revêtement polymère.

3. Composition selon l'une quelconque des revendications 1 à 2, dans laquelle l'absorbant ou l'adsorbant de dioxyde de carbone est soit sous forme granulaire ayant un diamètre de 0,1 à 10 mm, de préférence de 2 à 4 mm de US Pharmacopeia 26, soit pulvérisé d'une manière connue *en* soi ou dans laquelle l'absorbant ou l'adsorbant de dioxyde de carbone est sous forme liquide.

4. Composition selon l'une quelconque des revendications 1 ou 2, dans laquelle le caoutchouc de silicone est obtenu à partir d'un caoutchouc de silicone liquide dans laquelle ledit caoutchouc liquide est vulcanisé, de préférence soit sous forme de caoutchouc de silicone liquide catalysé par du platine en deux parties, soit sous forme de caoutchouc de silicone liquide en une partie avec un ou plusieurs initiateurs de peroxyde.

5. Composition selon l'une quelconque des revendications 1 à 4, dans laquelle la composition est utilisée par administration orale au moins une fois, de préférence au moins 3 fois par jour.

6. Composition selon l'une quelconque des revendications 1 à 5, dans laquelle l'administration orale est réalisée avec un comprimé, dans laquelle l'absorbant ou l'adsorbant de dioxyde de carbone est incorporé dans du caoutchouc de silicone ou avec une capsule, dans laquelle l'absorbant ou l'adsorbant de dioxyde de carbone est contenu dans une capsule faite de caoutchouc de silicone vulcanisé qui est de préférence scellée avec un revêtement de caoutchouc de silicone ou d'une cellulose insoluble dans l'eau.

7. Composition selon la revendication 6, dans laquelle le comprimé est choisi parmi des granulés, des mini-comprimés et des comprimés ayant un poids de 0,01 µg à 0,6 g, de préférence de 0,01 g à 0,1 g.

8. Composition selon l'une quelconque des revendications 1 à 7, dans laquelle l'administration orale est donnée à un mammifère qui est choisi parmi des ongulés comprenant des chevaux et des chameaux, des carnivores comprenant des chats, des chiens et des humains, dans laquelle les humains sont préférés aux mammifères.
